(19) Europäisches Patentamt European Patent Office Office européen des brevets

(11) **EP 4 483 810 A1**

(12) **EUROPEAN PATENT APPLICATION**

(43) Date of publication:
**01.01.2025 Bulletin 2025/01**

(21) Application number: **23182095.2**

(22) Date of filing: **28.06.2023**

(51) International Patent Classification (IPC):
***A61B 6/00*** (2024.01)

(52) Cooperative Patent Classification (CPC):
**A61B 6/482; A61B 6/481; A61B 6/507;**
**A61B 6/5217;** A61B 6/4441; A61B 6/486;
A61B 6/504

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **HAASE, Hans Christian**
**Eindhoven (NL)**
• **GRASS, Michael**
**Eindhoven (NL)**

(74) Representative: **Philips Intellectual Property &**
**Standards**
**High Tech Campus 52**
**5656 AG Eindhoven (NL)**

(54) **QUALITY CONTROL OF CONTRAST AGENT USE THROUGH DUAL ENERGY X-RAY IMAGING**

(57) A System (SYS) and related method for quantifying efficiency of use of contrast agent in an imaging procedure. The system (SYS) comprises an input interface (IN) through which is receivable input data comprising spectral projection imagery ($\lambda$) of an object (PAT) obtainable by a spectral X-ray imagining apparatus (IA) in an imaging procedure at a field of view, FOV, with contrast agent (CA) present in the FOV. An efficiency quantifier (EQ) computes, based on an amount of contrast agent estimable from the spectral projection imagery, a metric (m) indicative of the said efficiency of the said contrast agent use.

FIG. 3

**Description**

FIELD OF THE INVENTION

The invention relates to a system for quantifying efficiency of use of contrast agent in an imaging procedure, to a related method, to an imaging arrangement, to a computer program element, and to a computer readable medium.

BACKGROUND OF THE INVENTION

Spectral X-ray systems, like a C-arm system with a dual layer detector for example, enable more advanced imaging methods, in particular, but not only, during minimally invasive procedures.

When imaging for structures of poor radio-opacity, such as blood vessels, for example heart coronaries, etc., contrast agent is used to achieve better image contrast in respect of those structures.

Contrast agent quantification, blood to contrast dilution measurement, and the dynamic behavior of contrast agent are measures that can help extract quantitative information of blood flow from X-ray images. For example, blood flow volume, velocity, and distribution have broad physiological significance for vascular diseases like coronary artery disease and peripheral artery disease, etc. Such flow related diagnostic quantities/measures may be used to diagnose disease, plan procedures, and evaluate outcomes, etc. Use of spectral imaging systems, such as dual energy X-ray alone already provides improved options for contrast agent-based analysis of X-ray images.

For the above or other imaging purposes, contrast agent is widely used to image vascular structures during endovascular procedures for example or other structures of poor radio-opacity. Useful as contrast agent administration for the above-mentioned purposes is, there are risks: too liberal a use may lead to kidney damage or health problems. For patient safety, unnecessary and inefficient use of contrast agent is best avoided. An example of such inefficient use is contrast agent flowing to different parts of the body, which are not of interest for the current procedure.

Also, a too high or too low a contrast agent (such as Iodine) concentration in vessel areas of interest may cause resulting images to be unusable. Alternatively, the images might have also been usable with less contrast agent dose.

SUMMARY OF THE INVENTION

There may therefore be a need for improved contrast agent-based imaging with improved utility, in particular for enhanced and safe use of contrast agent in imaging procedures.

An object of the present invention is achieved by the subject matter of the independent claims where further embodiments are incorporated in the dependent claims. It should be noted that the following described aspects of the invention equally apply to the related method, to the imaging arrangement, to the computer program element and to the computer readable medium.

According to a first aspect of the invention there is provided a system for quantifying efficiency of use of contrast agent in an imaging procedure, comprising:

an input interface through which is receivable input data comprising spectral projection imagery of an object obtainable by a spectral X-ray imagining apparatus in an imaging procedure at at least one field of view, FOV, with contrast agent present at least at times in the FOV; and
an efficiency quantifier configured to estimate an amount of the contrast agent from the spectral projection imagery and compute a metric indicative of said efficiency of use of contrast agent, based on the estimated amount.

In embodiments the system may be operable whilst such imaging procedure is on-going.

In embodiments estimating the amount of the contrast agent includes integrating, spatially and/or temporally, over pixel values in one or more contrast agent-only-images derived from the spectral projection imagery acquired of the region of interest.

In embodiments the metric is based on the total amount of contrast agent used in a part of the imaging procedure, or in the whole of the imaging procedure.

In embodiments the metric is based on the amount used locally in at least one subset of the FOV, or in the whole of the FOV, as captured in the spectral projection imagery.

In embodiments the metric is further based on comparing the said amount against a predetermined amount of contrast agent. The predetermined amount may be provided by an automated or manually operable contrast agent adminstering device (pump, etc) which configured to measure the amount of contrast agent so released or administered.

In embodiments the said metric measures a suitability for the imaging procedure of the amount of contrast agent.

In embodiments the said metric measures a concurrency of a time period of the imaging procedure versus a time period in which contract agent is released into the FOV.

In embodiments the said metric is based on the number of times contrast agent is releasable in the imaging procedure.

In embodiments the said metric is configured to account for an imaging geometry for the imaging procedure.

In embodiments the efficiency metric (m) is computed based on a segmented portion in the spectral imagery, and the said amount is estimable relative to the said segmented portion.

In embodiments segmenting the segmented portions distinguishes between a first region of interest for the imaging procedure and a second region. The second region may be a "region not of interest", or a region of less interest, for the imaging, it is disjoint (void set-theoretic intersection) from the first region which is of interest for the imaging. The efficiency metric may then be based on a first amount of contrast in the first region, and a second amount of contrast agent in the second region.

In embodiments, the efficiency metric may be based, additionally or alternatively, on a deviation of the contrast agent amount from a pre-defined amount (C0).

In embodiments the metric is computed based on a filing curve that traces the amount of contrast agent over time. Filing curve may relate to whole image/FOV, or only parts thereof.

In embodiments the metric is computed based on integrating (summing, eg $\int p$, $\Sigma p$) over pixel values p in the spectral projection imagery.

In embodiments the said integrating-over includes integrating over time, that is, over multiple over time frames that form the imagery.

In embodiments, any one or more of: i) the segmented portion, ii) the region of interest, and iii) the second region, represent at least a part of at least one conduit in the object (PAT), at least a part of the said contrast agent being present in said conduit, at least at times.

In embodiments the conduit is part of a vascular, urinary or lymphatic system of a mammal, such as a human patient.

In embodiments the efficiency quantifier capable to compute an initial amount of the contrast agent upon entering the FOV and/or a rate of entering into the FOV of the said contrast agent, and/or at rate of exiting the FOV of the said contrast agent.

In embodiments the imaging apparatus is configured for vascular imaging.

In embodiments the spectral X-ray imagining apparatus is one of: the C-arm type, CT, configured for angiography, configured for fluoroscopy, the radiography type.

In embodiments the spectral imaging apparatus is configured for spectral imaging by virtue of any one of: i) having a detector configured for energy discriminative detection of X-ray intensities, ii) being configured for imaging with multiple, different, X-ray spectra. Dual energy imaging with two layers, or photon counting may be used. More than two layers may be used in N-energy imaging, N>2. Multi-X-ray source setups, or kVp switching, or filtering may be used instead.

In embodiments the spectral projection imagery includes a time series of frames.

In embodiments the spectral projection imagery includes a coronary angiogram.

In embodiments the computed metric is provided for processing, wherein the processing includes any one or more of: i) displaying on a display device an indication of the metric, ii) storing same in a data storage, iii) controlling operation of medical equipment, and iv) processing by a quality control system. Medical equipment may include any one or more of imager and contrast release device (eg, pump), or other equipment.

In embodiments, the efficiency quantifier is based on a trained machine learning model.

In another aspect there is provided an imaging arrangement comprising the system of any one of the above mentioned embodiments, and including the spectral X-ray imagining apparatus. The system may be integrated into the imaging apparatus, such as in its operator console, into any other associated computing device, workstation, etc.

In yet another aspect there is provided a method of image processing, comprising:

receiving input data comprising spectral projection imagery of an object obtainable by a spectral X-ray imagining apparatus in an imaging procedure at at least one field of view, FOV, with contrast agent present at least at times in the FOV; and

computing a metric indicative of said efficiency of use of said contrast agent, including estimating an amount of the contrast agent from the spectral projection imagery.

In another aspect there is provided a method of training the above-mentioned machine learning model, based on training data.

In yet another aspect there is provided a computer program element, which, when being executed by at least one processing unit, is adapted to cause the processing unit to perform the method or the method of training.

In yet another aspect there is provided at least one computer readable medium having stored thereon the program element, and/or having stored thereon the trained machine learning model.

The contrast agent may pass through and/or reside in the said conduit of system of such conduits. The conduit may be a blood vessel (such as coronary arteries or vein, or other), and the fluid may be blood including an admixture of contrast agent.

The proposed system may allow computing, based on the estimated contrast agent amount, the said metric or metric that quantifies efficiency of contrast agent use during a procedure. This metric may be used for quality control or for controlling medical equipment. Based on such an efficiency metric, future procedures, or overall staff performance, can be monitored and improved. In some cases, the so estimated amounts may be used as such as metric. Thresholding may be used to establish such a metric.

Spectral X-ray imaging, in particular in an interventional setting, can be used to derive such an efficiency metric. For example, some newer generations of C-arm X-ray systems are equipped with a dual layer X-ray detector. The front layer absorbs more low energy radiation and the back layer more high energy radiation. Due to the different spectral composition of the two 2D images (front and back layer image), different projection images at different energy spectra may be acquired for a given projection direction, and a spectral material decomposition of any 2D projection image can be calculated.

In particular a contrast-agent-only-image (such as for Iodine or whichever contrast agent material is used in the current imaging) may be calculated using a spectral imaging algorithm, which allows estimating an absolute quantification of the amount of contrast agent in the field of view (FOV) of the imaging apparatus as per the respective image, or in a specific region of interest (subset of the image).

Operation of the efficiency quantifier may thus broadly include estimating contrast agent amount(s), and then processing the estimated amount(s) to obtain the efficiency metric.

In many embodiments, the estimating of the contrast agent amount(s) may include integrating, spatially and/or temporally, over pixel values in one or more contrast agent-only-images (virtual contrast ("VC") image) derived from the spectral projection imagery acquired of the region of interest, in which contrast agent resided or resides during imaging. The amount of contrast agent so obtained by integrating pixel values, or in any other way, may be used to compute the metric.

The proposed metric may address a current clinical need for a measure of efficient use of contrast agent, which is currently not sufficiently monitored. Whilst an absolute contrast agent dose as released in patient may be recorded, this may naturally vary dependent on each patient. The manual logging of absolute contrast agent dose as released is an additional task that uses up much time of already time-pressed clinical staff. The proposed method and system allow collecting such a contrast agent efficiency metric automatically, more efficiently, more accurately, and adapted to the imaging task at hand. The efficiency of use may be measured dynamically over time, with the metric estimated as a time-varying quantity. Thus, the proposed efficiency metric facilitates safer operation of contrast agent-based imaging equipment.

Thus, in embodiments, the proposed system processes spectral X-ray projection image data obtained in a given contrast agent consuming imaging procedure to compute the efficiency metric that measures efficiency of use of the contrast agent during the said procedure. Whilst projection imaging is preferred herein, the metric may be also computed in angiographic CT (computed tomography), if required.

The X-ray projection images may be supplied by a dual layer C-arm system that delivers spectral intraprocedural images. Other spectral setups such as dual source, photon-counting, etc, may also be used. The C-arm imaging setup, whilst preferred for interventional procedures, is not a necessity herein, and any other suitable imaging hardware, for interventional or diagnostics use, may also be contemplated. Use of the system in MRI or PET/SPECT may also be contemplated.

The processing by the system may comprise computing an overall released contrast agent amount/dose, based on the spectral X-ray projection image data acquired throughout some part or all of the duration of the intervention.

The processing may include image segmentation to distinguish between contrast agent that enters a region of procedural interest and contrast agent that is lost in other areas of the body (e.g. contrast clouds / backflow during coronary injections) not of interest of the procedure. Thus, an amount (eg, amount, dose, etc) of contrast agent may be computed for/in two different image regions: a first region of procedural interest and, in addition, for a different, second image region outside the region of interest, in other words, an image region that is not of procedural interest or of less procedural interest.

That is, a first contrast agent amount may be estimated for the region of interest and a second contrast agent amount may be estimated in the region of no or less procedural interest. The two amounts of contrast agent may be algebraically combined such as by forming a ratio or difference or other between the two, based on which the efficiency metric may be computed. Such ratio or difference, for example, is indicative of a portion of contrast agent that has flowed or leaked into regions of less or no interest, which may be harmful to a patient. Thus, in some embodiments, such ratio or difference itself may be taken as the metric.

In some embodiments, a local contrast agent amount in a specific region(s) of interest is computed, and compared to a pre-defined amount (e.g. a threshold) to establish a deviation. The metric may then be computed as a function of the deviation, or the deviation may be used as the metric. The two (or more) metrics may be combined to derive another type /version of the metric, etc.

The pre-defined amount may be a generic value or a patient-specific value. In the latter example, the patient-specific value may be determined or calculated based on one or more parameters retrievable from an electronic health record.

In another example, a predetermined amount of contrast agent may be measured by an automated contrast agent pump or other contrast agent release device and used for comparison with the estimated contrast agent amount, on which comparison the metric may be based. In other words, the pre-defined value of an amount of contrast agent corresponds to a reference measurement of an amount of contrast agent being provided by such contrast agent administering device.

For example, if, according to the reference at pump, 30 ml of contrast agent was released, but only 10 ml was estimable in the input image imagery, this shortfall may be an indication of inefficient usage. A deficiency of 20 ml may mean that a sizable amount of contrast agent got "lost", e.g. by accumulation in other body parts. The deficiency may be expressed as %-value of the initially released amount (eg, 30 ml). The numbers given in this paragraph and elsewhere are merely for illustration and in no way limiting to the principles described in this paragraph and elsewhere in this disclosure.

The contrast agent amount (e.g. mass) extraction/estimation may be done by pixel value integration, with or without prior filtering and/or vessel tree segmentation. Integration may be over time (frames) and/or spatially, over pixels/voxels, in one (or more) frames.

A fluid, in which the contrast agent is entrained or dissolved therein, passes through a conduit, such as pipe or tube system or similar, and is distributed therein by the flow of the fluid that carries the contrast agent. Blood flow through vessels, such as arterial or veinal coronaries in the human heart, or through other blood vessels of other organs/anatomies are mainly envisaged herein. However, other conduit structures such as lymphatic systems, urinary, gastric, etc., are not excluded herein, and neither are non-medical applications such as examinations based on released dyes or similar, entrained in a fluid, such as in water as in speleology or hydrology, or in examinations of inaccessible plumbing systems, of hydraulic system or of other pipework in machine parts in vehicles for example, or in any other type of machinery, etc.

"User" relates to a person, such as medical personnel or other, operating the imaging apparatus or overseeing the imaging procedure. In other words, the user is in general not the patient.

"Metric" may be any numeric value or values, in scalar, vector or matrix form. The metric is configured (computed) so that it responds to/varies with efficiency of contrast agent usage. The metric may be binary (efficiency, yes/no), or a continuous value relative to a scale.

"Contrast agent amount" may be expressed in any suitable form such as volume (eg, ml), mass (eg, mg), concentration, in-tissue path length of line integral, etc. It may be express in absolute or relative terms.

"Spectral imagery" may include single frame (acquisition) or multiple such frames over time. Spectral imagery is combined by spectral imaging algorithm (such as material decomposition, or other) from plural projection images acquired at different energy spectra. The spectral imagery may be in projection domain (spectral projection imagery, mainly envisaged herein), or may be in image domain, such as tomographic reconstruction from spectrally processed plural projection images. Spectral imaging allows imaging at material specific contrast, or for contrast that are specific for a conglomerate of material, or to contrast at the exclusion of such specific material or conglomerate of materials.

"Contrasted (projection image)"/"un-contrasted (projection image)" is shorthand for projection raw imagery acquired by the imaging apparatus whilst there is, or there is no ( or only negligible) contrast agent within the FOV (field of view) or the imaging apparatus, respectively.

"FOV" relates of a portion of or 2D or 3D space (the exam region) imaged, with at least part of object therein.

BRIEF DESCRIPTION OF THE DRAWINGS

Exemplary embodiments of the invention will now be described with reference to the following drawings, which, unless stated otherwise, are not to scale, wherein:

Fig. 1 show a block diagram of a medical imaging arrangement as may be used in particular to support contrast-based imaging;

Fig. 2 shows a schematic drawing of a conduit system, such as a vessel system, in which contrast agent is residing therein;

Fig. 3 shows a block diagram of a computerized system for computing a contrast agent usage efficiency metric;

Fig. 4 shows filling curves as may be computed herein in facilitating the computation of contrast agent amount based on spectral projection imagery;

Fig. 5 shows in-image regional designators that may be used for computing an efficiency metric of contrast agent's usage in a procedure according to one embodiment;

Fig. 6 shows a graphics display generatable herein according to an embodiment as envisaged herein;

Fig. 7 shows a graphics display generatable herein according to another embodiment, including a representation of a time curve of efficiency metric for contrast agent usage in a procedure; and

Fig. 8 shows a flow chart of a method of computing a metric of efficiency of contrast agent use, based on spectral imagery.

## DETAILED DESCRIPTION OF EMBODIMENTS

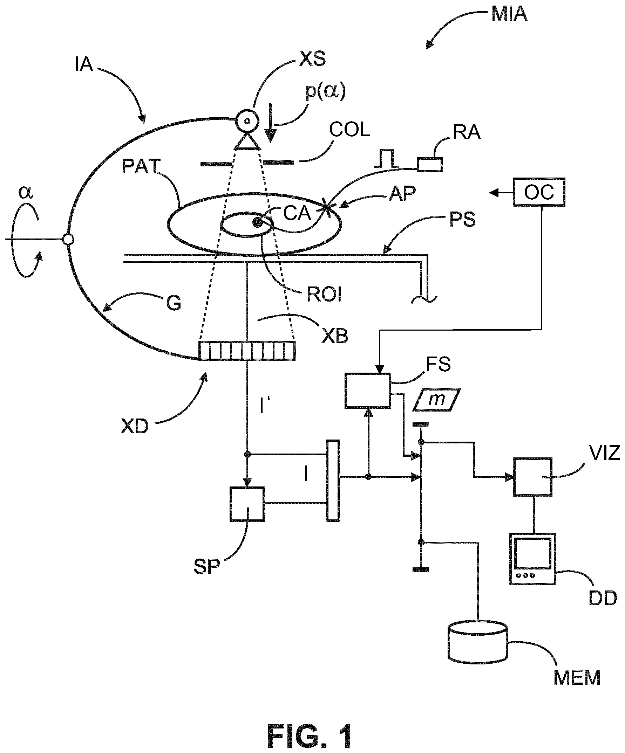

Referring first to Fig. 1, this provides a block diagram of a medical imaging arrangement MIA configured to facilitate efficient use of a contrast agent in a fluid during contrast agent-based imaging procedures, such as of the interventional type, but non-interventional applications are not excluded herein.

What is proposed herein in embodiments is a computing-based facilitator system FS (referred to hereinafter as "facilitator FS"), capable of supporting a contrast-agent based imaging procedure of any imaging apparatus, preferably of the X-ray based type and configured for spectral imaging for good results. The imager IA is preferably further configured for fluoroscopy /angiography. The facilitator FS is capable of computing an efficiency metric m (referred to herein also as "e-metric") for the efficiency of the use of contrast agent in the imaging procedure. The computational approach herein is carried out preferably during ongoing imaging procedure and(/or contrast agent release. The facilitator may be used in image-guided interventions, such as in relation to the mammal brain or heart, or any other organ/anatomy, as needed. The imaging procedure may be based on a stream (time series) of spectral projection imagery $\lambda$, in a dynamic setup where contrast agent accumulates or depletes over time. Time evolution of such accumulation is recorded.

The computation by facilitator FS's efficiency quantifier component EQ (to be explained in more detail below) of e-metric m can be purely image based as mainly envisaged herein. However, contextual control parameters of the imaging procedure may be gotten by interfacing with the operator console OC, or other component of the imaging apparatus or other component such as contrast agent release apparatus RA, and such option is also envisaged herein.

Efficiency of contrast agent use as measured by the e-metric is preferably based on the amount of contrast agent used during the intervention, specifically during the imaging (such as X-ray exposure) or even outside the imaging period T. More specifically yet, it is the amount of contrast agent that finds its way into a pre-defined region of interest ROI on which the efficiency metric may be based. Thus, the e-metric is configured to respond to such local usage and may dynamically vary with changed usage, in particularly and solely in relation to the use (amount present) in the pre-defined, dedicated, ROI for the imaging procedure. Such imaging procedure may include assessment of a stenosis, placement of a stent, check of such or prior placement, heart valve repair, embolus removal, angioplasty, or examination or manipulation in relation to vessels in the brain of patient PAT, etc.

Such local, ROI-focused response of the computed e-metric can be refined by making this metric respond not only to the amount of contrast agent in the ROI, but also in addition to how it is used over time for example, and/or in relation to the specific imaging procedure used, its parameters (imaging geometry, beam control (collimation, tube amperage/-voltage), etc. The imaging procedure may be defined by imaging protocol. The imaging protocol may prescribe imaging or other parameters that may be used herein to compute or refine the efficiency metric.

The proposed set up harnesses the material specific contrast conferring power of spectral imaging. Spectral imaging is an imaging technique that is partly hardware and partly computational based. Multi-channel (multi-energy) in spectral imagery acquired due to a specific imaging hardware set up is computationally combined to obtain the material specific imagery. A computation may based on prior knowledge of the material specific attenuation co-efficient (material property) of the material(s) of interest. Such attenuation co-efficient are known and can be obtained from lab tables or other material reference works.

Before explaining operation of the facilitator system FS in more detail, reference is now made first to the general imaging setup as may be used herein in the medical imaging arrangement.

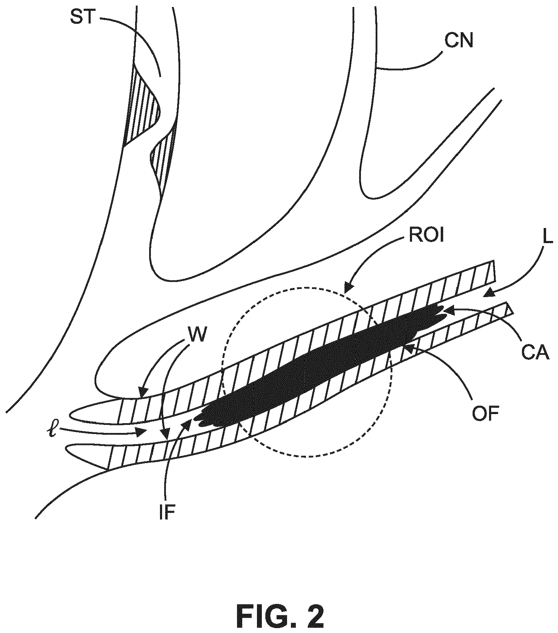

The medical imaging arrangement MIA may include a, preferably, X-ray based imaging apparatus IA (also referred to herein simply as "imager"). The imaging apparatus IA may be of the C-arm/U-arm type so allows collection of projection imagery $\lambda$ along different projection directions $P(\alpha)$ around the region of interest, such as around a stricture ST in a coronary vessel (see Fig. 2 below).

The imaging apparatus IA includes an X-ray source XS and an X-ray sensitive detector XD. In some embodiments, the X-ray source XS, and opposite thereto, the X-ray sensitive detector XD, are arranged preferably on a rotatable gantry G. The gantry G may have a recess in which an examination region is defined where the patient, or in particular the region of interest, resides during imaging. The rotatable gantry may thus have a "C", "U" or similar shape, such as in C-arm imaging apparatus envisaged herein. However, such or similar rotational or an otherwise dynamic setup, where there is at least some relative motion between source and detector intended, is not necessarily required herein in all embodiments. Instead, a planar radiographic setup is also envisaged in some embodiments. Instead of using a single detector as illustrated in Fig. 1, a bi-planar imaging setup with two detectors with intersecting imaging axes (for example at right angles) may be used to obtain and image for at least partial 3D information in relation to ROI.

In addition, whilst digital-at-source imaging, such as by flat panel detectors is preferred, where the imagery is natively acquired as digital data, such as setup is not necessarily required. More traditional, cassette-operable, analog imaging methods (based on X-ray films) are not excluded herein. The analog imagery may be digitized post-acquisition for example, by using a digital camera. Imaging based on X-ray image intensifiers, etc with post-digitization is also envisaged in some embodiments.

Generally, imaging includes energizing the X-ray source XS, so that an X-ray beam XB issues forth from a focal spot of the source XS, traverses the examination region (with the region of interest in it) and interacts with patient tissue matter. Interaction of X-ray beam XB with tissue matter causes the beam to be modified. The modified beam may then be detected at the detector XD. Conversion circuitry (not shown) converts detected intensities into digital imagery, in particular into projection imagery. The imagery may be used by user in the medical procedure. The patient may reside on a patient support PS such as a table during imaging. Soft tissue such as the one making up the coronary or other vessels, provide usually poor contrast in native X-ray imagery. In order to boost contrast, the mentioned contrast agent (sometimes "CA" or short), such as an iodine solution, gadolinium, or other, is delivered through an access point AP into the blood flow of the patient prior to or during imaging.

The contrast agent CA may be delivered via a CA release apparatus RA, such as a motorized pump, but manual delivery is not excluded herein by way of a syringe of some such. The CA release apparatus RA may be operable to deliver a defined volume ("bolus") of contrast agent to patient PAT. The volume of contrast agent so delivered is entrained with and thus travels along with the blood flow as one example of a fluid in a system of conduit CN (vessels) envisaged herein. After a certain period of time, contrast agent accumulates at the region of interest, such as in or around the stricture or other lesion. Image acquisition is preferably synchronized with arrival of contrast agent at the ROI. Preferably "contrasted" imagery is acquired, that is imagery is so acquired by exposure of X-ray whilst there is contrast agent CA present at the ROI, or at least in the current FOV. The FOV may be changed, by operating a collimator COL to tighten the X-ray beam XB, by moving gantry G to shift FOV, or by repositing X-ray source XS, to so select different projection directions $\alpha$, along which imagery is acquired. Optionally, additional "non-contrasted" imagery is acquired whilst no or negligible contrast agent is present at the ROI.

The in particular contrasted imagery may be acquired in a time series with varying amounts of the contrast agent accumulating at the region of interest ROI. Image acquisition may start before, may continue throughout, and may terminate after accumulation of the contrast agent at the ROI. In general, contrast agent amount increases over time after delivery, will plateau at a certain maximum value, and will then wash out or decrease over time until completely vanished. Whilst a single or certain discrete number of still images may be acquired in some instances, acquisition of a stream images, or frames, acquired at a suitable frame rate is preferred as this allows real time dynamic observation. Specifically, the acquired stream of projection imagery may be visualized by a visualizer VIZ as a live video feed on display device DD. Alternatively or in addition, still imagery is so visualized. Operation of the facilitator system FS is preferably based on processing such, as still or stream imagery. The imagery may be stored in an image memory MEM or may be processed otherwise.

X-ray imaging is generally based on the attenuation co-efficient of the matter(s) that makes up the region of interest. The attenuation (coefficient) is dependent on the energy of the X-ray beam. In order to harness this energy dependency, the spectral imager IA may be used, configured for spectral imaging. This may include detector-sided or source-sided solutions. Detector-sided solutions include photon-counting detector hardware or a dual-energy setup with dual-layer hardware for example. More than two detector layers may be used although this is less common. Source-side solutions include multi-source setups, kVp switching, filtration and other.

Such an imaging set-up in spectral imaging allows acquisition of multi-dimensional projection raw data $\lambda'$. Such multi-dimensional image data includes projection imagery at different energy levels/windows or spectra more generally E1, E2 (such as two such levels or spectra in dual imaging, but more than two spectra may be used in corresponding setups) per projection direction $\alpha$. Thus, for each exposure or projection direction $\alpha$, there are multiple projection images obtained, at least one such projection image per energy level/spectrum E1, E2: $\lambda' = \{\lambda'E1, \lambda'E2\}$. Thus is in distinction to acquiring energy integrated imagery as in a conventional (non-spectral) X-ray imaging set-up, the latter not being excluded herein. For example, such a conventional set-up may still be used herein for digital angiography subtraction ("DAS") where pairs of projection images at higher and lower amount of the contrast agent are acquired. Such image pairs are then optionally spatially registered and subtracted from each other to boost contrast of the soft tissue including the contrast agent in the difference image to obtained. DSA may also be practiced with spectral imaging setup, and such is indeed envisaged herein in some embodiments.

Preferably however, for more accurate/direct quantification of the contrast agent amount at the region of interest, a spectral imaging set-up is preferred. Such a spectral imaging setup further includes a spectral image processor SP, in addition to the above described hardware arrangement for acquiring the projection raw data $\lambda'$ at multiple spectra E1,E2. The spectral image processor SP may implement a spectral image processing algorithm, such as material decomposition or other. Broadly, the spectral image processor SP is operative to combine the different projection images $\lambda'E1$, $\lambda'E2$ acquired by exposure to radiation at such different spectra E1, E2, to so obtain spectral projection imagery $\lambda$, and such will be mainly considered herein as input for the proposed facilitator FS.

A range of such spectral image processing algorithms are envisaged herein. Specifically, the spectral processor SP processes the multi-energy (raw) projection imagery λ'E1, E2 acquired by the source- or detector-sided imaging set-up just described, and computes therefrom spectral imagery λ that differs from the original multi-energy raw projection imagery λ' = {λ'E1, λ'E2}. Spectral imagery λ is thus computationally derived from the input multi-energy projection imagery λ'. Such spectral imagery, which may be in projection domain, may include for example, a monochromatic image at a desired energy level, or a virtual non-contrast image ("VNC"), or a virtual contrast-only image ("VC" - e.g. Iodine image), a Compton scatter image, and others.

The monochromatic image may correspond to the attenuation co-efficient of the contrast agent used. For example, the energy value or energy range for the monochromatic image may be chosen to equal or include the K-edge energy of the particular contrast agent to be used for good contrast for example. However, such as K-edge based choice is not mandatory herein and other energy values or ranges for the mono-chromatic imagery is equally envisaged herein. The monochromatic image represents a computational approximation of an image that may have been obtained had a monochromatic X-ray source XS at the respective energy level been used for imaging. In most practical cases, the actual X-ray source XS is polychromatic.

The VNC image represents an approximation of an image that may have been obtained if there had been no contrast agent present in the field of view of the imager IA, at the exclusion of other materials.

The VC image, and of main interest herein, represents an approximation of an image that may have been obtained if there had been only contrast agent present in the field of view of the imager IA, at the exclusion of other materials. Thus, image contrast in VC is exclusively or pre-dominantly contrast agent focused. It is preferably the spectral (projection imagery) so computed by spectral processor SP that is processed by the facilitator system SYS, instead or, or in addition, to the original multi-energy input projection imagery, as will be described below in more detail.

For simplicity, the designation "λ" may be used herein to indicate input spectral imagery in general to be processed by the facilitator system FS. That is, "λ" may relate to the spectral projection imagery as computed by the spectral processor SP based on the original projection raw imagery/data λ' as acquired. The spectral processor SP and the facilitator system FS may be implemented on different computing systems, possibly geographically separated such as in a distributed "cloud" architecture. However, implementation on a single computing system is also envisaged herein.

Imaging as envisaged herein is preferably attenuation-based, but this is not at the exclusion of other X-ray modalities, such as dark-field imaging and/or phase contrast imaging. Whilst tomosynthetic or tomographic imaging is not excluded herein processing of projection imagery solely in projection domain is mainly envisaged herein in embodiments. Whilst not necessarily used for tomographic imaging, the imager IA may still be capable for multi-directional imaging in a rotational setup such as the C-arm as described, so as to collect protection imagery from the best clinically mandated angle α (which may change in the procedure), or at least to be able to collect some at least partial 3D information in relation to the ROI. However, such rotational setup for multi-directional imaging is not necessarily required herein. Instead, imager IA may have a fixed, or at least-non-rotational, frame G, such in a bi-plane arrangement, specially envisaged herein in some embodiments.

Thus, the spectral processor SP may thus operate in projection domain. However, reconstruction from projection imagery into sectional or volumetric imagery by tomographic reconstruction using a reconstructor component (not shown) is not excluded herein. Thus, in this embodiment, λ may relate to spectral VC reconstructed volume or slice imagery. But as said, imaging in projection domain is mainly envisaged herein. Thus, the input imagery λ considered herein is mainly a spectrally processed VC projection imagery, either single frame, or multiple over time frames. Processing by facilitator FS into e-metric m can be done frame by frame, or some or each processing by facilitator FS may include processing multiple frames to obtain e-metric at a given time t.

Reference is now made to Fig. 2, which shows a schematic representation of a situation that may be encountered when imaging conduits CN through which fluid with contract agents CA passes and as envisaged herein in embodiments. In preferred embodiments, the conduit (system) CN may include an arterial or a veinal vessel system, such as of the human heart, the brain, or of other organ. As indicated in the upper branch of Fig. 2, a stenosis ST may narrow lumen L of the vessel system. Such stenosis may be due to a build of deposits such as plaque in wall W of vessel CN. Such stenosis may lead to health problems. The correct identification of such a stenosis, its severity, or other aspects thereof, can be done by an image-based examination of the flow characteristics of the blood in the affected vessel CN. Mixing into the blood of contrast agent confers better imaging contrast of the lumen's spatial structure as the blood-contrast agent admixture passes through the vessel CN and fills the said lumen, thanks to the contrast agent's radio-opacity and its liquid state in the admixture, assuming the shape of the confines of the vessel where it resides.

Into is only for clarity of illustration, that the amount of contrast agent CA ("bolus") is shown in a different part of the vessel tree CN than is the lesion ST. However, in reality, the blood with the CA therein is to propagate into and possibly through the stenosis ST site. The stenosis ST can thus be detected visually by user, or (semi-)automatically by an image recognition component, as needed and depending on the clinical task at hand.

With continued reference to Fig. 2, this affords a sectional view of such a vessel system including a branch through which contrast agent CA is passing with the blood flow along the length ℓ of the respective branch. A region of interest ROI "shown in dashed lines may be geometrically defined either automatically (eg, by segmentation) or user prescribed (by using a pointer tool for example). Again, this ROI is to include the site of the stenosis ST and the contrast bolus CA, and it is only for clarity of graphical illustration that lesion ST and ROI/contrast agent are shown in different portions of the vessel CN.

The region of interest ROI may be demarked by a graphical user interface on the acquired imagery and/or may be defined by collimator action, etc, as required. The contrast agent amount estimation by facilitator FS as described herein may be related to the said region of interest ROI. Thus, a main interest herein is the estimation by computation of contrast agent CA amount in the part of the vessel CN as defined by the ROI. The ROI may relate to a sub-part of the vessel system CN such as shown in Fig. 2, such as to one or more branches (with or without its collaterals), or may relate to the whole imaged vascular system CN in the current field of view. Thus, the contrast agent amount (such as concentration) of interest herein refers to the amount of contrast agent CA, such any one of number of particles, volume or mass of contrast agent in a reference volume, concentration, etc, as defined by the region of interest ROI in the respective vessel CN.

In some cases, it is the amount of contrast agent CA in the lumen L that is of main interest. Lumen L represents the (whole) space inside the vessel CN surrounded by vessel wall W. In some, but not all embodiments, it is the volumetric concentration or mass concentration of contrast agent CA in respect of the section of the lumen L inside ROI that may be computed herein for the purpose of CA efficiency of use. For example, mass concentration may be defined as the ratio of mass of contrast agent by the lumen volume L in the ROI considered. The contrast agent concertation may be essentially zero, when there is only blood present in the ROI, such as prior arrival of the initially released amount of contrast agent (bolus) as it travels with the blood flow. At the other extreme, the concentration may be such that there is substantially no blood present, such as at time when contrast agent displaced all blood in the ROI considered. The contrast agent amount will be understood to vary over time. Thus, contrast agent amount may relate to the current amount at a given instant/frame, or may relate to an average of contrast agent amount readings/values over time, or to another such consolidated quantity that combines contrast agent amount information across frames. The spatial extent of the lumen L may be estimated if the concentration of contrast agent is to be computed. Such estimation may be done by suitable spatial imaging, such as along different projection directions, or by spatial modeling, or clinical knowledge or any other way. However, such spatial estimates of lumen L, or contrast agent amount in terms of concentration in not needed in all embodiments herein. Total contrast agent mass estimates may be sufficient herein in preferred embodiments.

The region of interest ROI may further allow defining an inflow portion IF where blood (including the contrast agent) passes into the region of interest, and an outflow portion OF where the said blood and contrast agent mixture egress the region. As will be explored in more detail below, definition of such in- and outflow portions IF, OF may be used herein to define one or more quantities of interest that measure how efficient contrast agent is used for a given image exam.

Although other applications of the principles described herein in medical and non-medical applications in support of image-based examination of conduit systems are also envisaged herein, we will be referring herein mainly to the conduit CN as a blood vessel CN such in the heart brain, lung, leg, arm, neck. etc, with the understanding that the said other applications are not excluded herein. Also, the contrast agent CA will be referred to herein at times as Iodine, although it will be understood that other radiation-opaque substances such as Gadolinium, a mixture thereof, or other substances can also be used herein.

Whilst X-ray imaging is mainly contemplated herein, other imaging modalities such as MRI, nuclear imaging (PET/SPCET), or others still are not excluded. In such other cases, the contrast agent use there may act differently and may have other suitable properties than increased radio-opacity, in order to suitably confer better image contrast in such other imaging modalities.

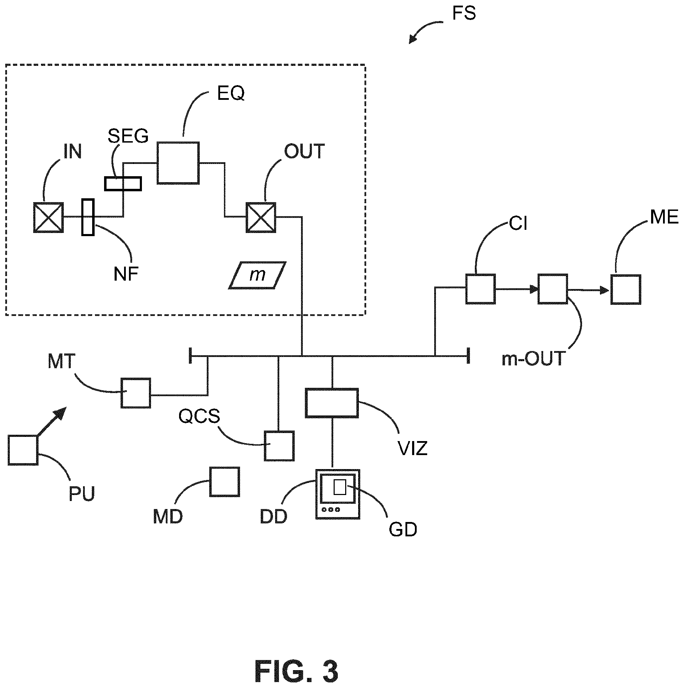

Reference is now made to the block diagram of Fig. 3 which shows components of the system FS for facilitating computing, estimating, or otherwise quantifying efficiency of use of contrast agent CA in contrast agent-based imaging. Fig. 3 illustrates in more detail operation of facilitator FS. Specifically, the system FS may facilitate determining the efficiency of contrast agent use in a region of interest ROI, such as in a vessel or section thereof.

Input spectral imagery $\lambda$, either a single still image or a time series of frames $\lambda = \lambda t$, is receivable at input interface IN. Also, the input imagery may be acquired along different projection directions or along a single one. The spectral imagery is in projection domain as mainly envisaged, but reconstructed sectional/volumetric input imagery reconstructed from such spectral projection imagery is not excluded herein. The input spectral imagery includes at least one (preferably more) frames spectrally computed as VC imagery as explained above, such as VC projection imagery. Although spectral imaging is preferred herein, this is not a requirement herein in all embodiments. DSA (digital subtraction angiography) may be used instead with conventional energy integrating projection imaging. However, as said, the proposed system FS may make its benefits best felt when using spectral imaging. DSA may also be used in spectral imaging, and such as envisaged herein.

The input spectral imagery $\lambda$ may be supplied by the spectral processor SP as the VC image, based on contrasted projection raw data as acquired at multiple energy spectra during imaging. Thus, contrast in the input spectral imagery $\lambda$ is assumed herein to be predominantly, if not exclusively, caused by attenuative contributions from contrast agent material present in the FOV during imaging, whilst (X-ray) attenuative contribution, and thus image contrast, from other materials that happen to be in the FOV during imaging may be assumed herein as negligible.

As in all imaging set ups, it also the spectral imaging that may be subject to image noise. To combat image noise, the facilitator FS may include a noise filtering NF component. In some embodiments, the facilitator FS may be implemented as a thresholder. Thus, some non-zero image signal contributions, but less than some threshold, may be recognized herein as rogue values that are responsive to noise contributions, rather than being indicative of contrast agent amount. Facilitator FS's noise filter NF may be operative to estimate noise based on a noise model. A suitable threshold based on the noise model may be defined. On whichever basis the threshold is configured, it is in generally only pixels beyond this noise threshold that are then taken into consideration for the integration, or for computing by efficiency quantifier EQ of the e-metric. The noise filter component NF may be based on any noise filtering algorithm, such as on using Gaussian or other kernels. In either case, it is the so noise filtered spectral projection imagery that is used to compute the e-metric. The filtering component NF may, in addition or instead, perform DSA by subtracting contrasted spectral imagery VC from non-contrasted spectral imagery ("VNC", virtual non-contrast), thus performing another form of filtering or conditioning, for yet better definition and detection of contrast agent contribution. When using the same projection direction, the contrasted spectral projection imagery is considered natively registered to their non-contrasted spectral projection imagery, thus enabling good quality DSA. Otherwise, an image registration may be needed.

The processing by efficiency quantifier EQ of facilitator FS to compute e-metric m envisaged herein can be done on a still image, on a given single projection image, but is preferably based on a time series of such spectral imagery frames, indicative of the evolution of the amount of contrast agent over time, as mentioned earlier. There may be single metric or a corresponding time series mt for such an e-metrics mt corresponding to each frame or a group of frames, etc.

In addition or instead to such noise filtering, a segmentor SEG may be used. The segmentor SEG may implement any segmentation algorithm to compute a segmentation s= s(Z) for contrast contribution. The above-mentioned integration by EQ over space and/or time t is thus not only confined to non-zero or non-negligible (thresholded) image values, but is further confined to such contrast agent segmented values, if segmentation is used.

The segmentation by segmentor SEG is preferably configured to represent the region of interest ROI in the current field of view, in particular a part of interest of the vessel structure in which the contrast agent resides, and which part is the object and purpose of the imaging as prescribed by applicable imaging protocols. In this manner a localized more improved, focused, contrast agent efficiency metric can be computed by efficiency quantifier EQ.

As mentioned, spatial and/or time integration of optionally filtered and/or segmented pixel values is used herein by efficiency quantifier EQ in some embodiments to estimate ("extract") the pertinent in-ROI contrast agent amount. The e-metric may then be computed based on the CA amount so extracted. The spatio- and/or temporal integration by efficiency quantifier EQ may be implemented by conditional looped summation which may be written formally as:-

$$m = \sum_{j \in U \subset \lambda} pj$$

$$(1)$$

$$m = \sum_{j \in U \subset \lambda_t, t \in T} p_j^t$$

$$(2)$$

With $p_j$, $p_j^t \in \lambda$, $\lambda t$, respectively, and U a qualifier subset of all locations j of pixel values pj or pjt, which are considered in the integration, and T a section or the whole of the period/duration of the imaging procedure, with t the various imaging instants. $U = \lambda$, $U = \lambda t$ in some instances, for example in applications with very tightly defined collimation by optional collimator COL, but in general qualifier subset U is less (a true subset) of the given image instance/frame $\lambda$, $\lambda t$. Qualifier subset U represents any one or all of filtered, thresholded, and segmented pixel values, in general any pixels that are qualified to be considered as true contrast agent signals.

Depending on U, T, the integrated value (1), (2) is representative of the total contrast agent used in part or the whole of the ROI in the FOV (optionally confined to the segmentation of the anatomy of interest) and/or over the whole or part of the imaging procedure. The integrated amount (1), (2) may be compared to a pre-defined contrast agent amount, such as threshold. Based on the comparison, e-metric is output at port OUT. The metric may be binary "0"/" 1 "(such as efficiency yes $\cong$ "1"/ or no $\cong$ "0") or may be a continuous value against a bounded scale such as [0,M], eg, M =1, with a suitable coding, such as a higher value being indicative of better efficiency, or vice versa as implementation, system embedding or convenience may call for. An indication of the computed e-metric may be output for storage, display, etc. A graphical representation GD or a textual /numeric token may be overlayed of the/or the current image and concurrently displayed on displayed device DD. The e-metric value m may be updated in a dynamic graphics display GD as the imaging procedure progresses and as the e-metric is being re-computed by efficiency quantifier EQ according to distribution/dynamics of the released contrast agent as recorded in the imagery $\lambda t$ over time t. The graphics display GD may be generated by visualizer VIZ.

The pre-defined contrast agent amount /threshold may be the amount initially released by a release device RD, such as an automated (or manually) operable pump (syringe).

The integration (1), (2) as mentioned may be extended across time t over multiple frames, in particular all frames, used in the intervention/imaging procedure, to so obtain a (total) accumulated contrast agent amount up to such time t. The time evolution thereof may be represented as a filling curve.

Whilst some such total or local integration of spectral image values (over the whole period T or parts thereof) with optional comparison against a pre-determined amount of initially released contrast agent is envisaged herein in embodiments, further refinements or alternatives to compute the e-metric m by efficiency quantifier EQ are also envisaged herein, which will be now explained in more detail.

Broadly, the following configuration types of e-metric calculation by efficiency quantifier EQ may be envisaged herein in different embodiments:-

    a) Global contrast agent usage
    b) Local contrast agent usage
    c) Suitability of contrast agent amount (eg, concentration)
    d) Timing of X-ray acquisitions
    e) Selection of X-ray settings
    f) Positioning of X-ray system

A single one, or more than one, of the e-metric types a)-f) may be computed herein in various embodiments. Each type of e-metric a)-f) may be said to relate to different aspects of contrast agent efficiency of use. Each type of e-metric may be configured to respond to (vary with) different such contrast agent usage efficiency aspects.

Broadly, the following metric type categories may be envisaged herein. For example, e-metrics of type a)-c) may be said to relate to spatial or temporal aspects of contrast agent, whilst e-metrics of type d)-f) relate to operational or control aspects of the imaging apparatus (eg. Imaging geometry, imaging settings, etc) or other equipment such a contrast agent releaser RA in relation to contrast agent. It will be understood that the types a)-f) of e-metrics listed above are not exhaustive but are merely exemplary. Other e-metrics than the below described may be configured and that may be said to fall within such type or broader categories. Some metrics may fall in precisely one such type or category, whilst others may fall into more than one type and/or category. As said, e-metrics of more than one type or category may be computed herein and may be output for display, may be used for control purposes, etc. Plural such e-metric of different types may be combined herein to form a hybrid type category of e-metric. The e-metrics of different types/categories may be mapped to the same scale and may then be combined, such as averaged, weighted averaged, etc. Each metric type or a hybrid type/category may be displayed over time as a metric time curve mt, to illustrate the evolution of different aspects of efficiency of usage of contrast agent. Some embodiments may be more confined, thus may compute and output only a single e-metric type/category. Computing and outputting time evolution mt of e-metric m is, whilst preferred, not a necessity herein. A single momentary snapshot value of e-metric m may be output instead, or an average of such e-metric (hybrid or not) may be output once the imaging procedure, or a phase thereof, concludes, etc.

The types of e-metrics a)-f) envisaged herein are now explained in more detail. For example, type a) of e-metric may be said to relate to the mentioned global contrast agent usage. This type of metric is configured to respond to (vary with) how much contrast agent was used in total in period T in the imaging procedure, or parts thereof t<T.

Type b) of e-metric may be said to relate to the mentioned spatial/local contrast agent usage efficiency in a given ROI or region of procedural interest/target area. Thus, this metric may help answer the question whether (all of) the contrast agent released into patient reached the target area /ROI.

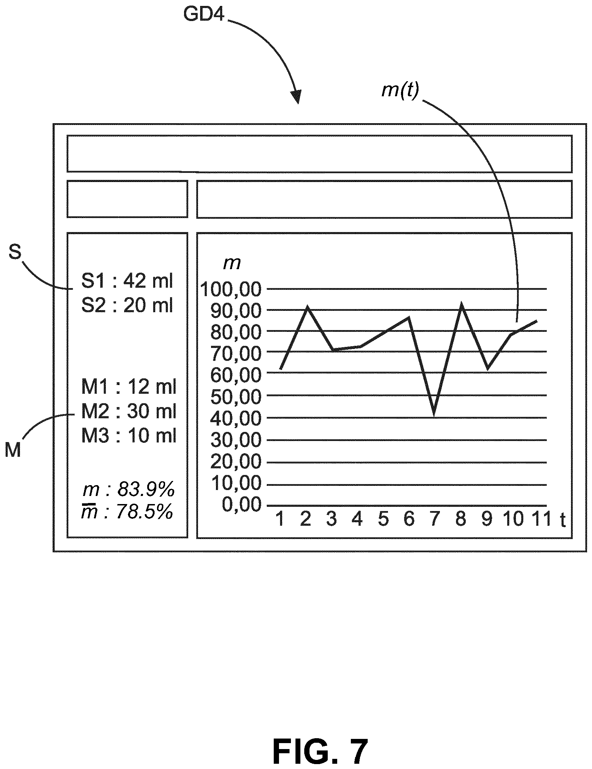

Type c) of e-metric may be said to relate to questions on concentration/dilution of the contrast agent amount: such an c)-type e-metric my thus help clinical user to answer questions whether the contrast agent was diluted too much or too little for example, or if the injection rate of the contrast agent was to high or too low (see the dashboard like graphics display at Fig. 7 , on which more further below).

Type d) of e-metric may be said to relate to imager control aspects in relation to control of contrast agent release apparatus RA. For example, such a metric may help answer the clinical question as to what extent the image acquisition (imaging) was synchronized with the contrast agent release. For example, did such image acquisition start and/or end with contrast agent release by release device RD.

Type e) of e-metric may be said to relate to the selection of imaging parameters in relation to the interrogative signal used, such as the X-ray beam to which ROI/patient is exposed to during imaging. For example, this e-metric of type e) may relate to X-ray settings. This e-metric may pertain to clinical questions such as "How often was contrast agent used in fluoroscopy, DSA, CT (cone beam, or other), etc.?" Thus, the e-metric may respond to such frequency.

Type f) of e-metric may be said to relate to positioning aspects of the X-ray beam (direction $\alpha$, collimation, shift of FOV (gantry, etc) relative to the contrast agent bolus, etc). Thus, type f) e-metric may relate to positioning of X-ray system IA/beam XB, generally. This e-metric may pertain to clinical questions such as "How much overlap (under the selected projection direction $\alpha$) of contrast filled structures was detected ?" Thus, the e-metric may respond and vary to various levels of such overlap.

A monitoring system/device MT may be used for monitoring the computed e-metric value(s). This may be done to ensure efficient use of contrast agent as an undue release thereof may itself lead to health problems for patient. The monitoring system MT may use thresholding. If the metric corresponds to inefficient use (as may be established by thresholding), an alert signal may be issued to alert the user. The alert signal may be visual, audio, haptic or a combination of any one of the foregoing. If there is efficient usage concluded, the system may remain unresponsive/dormant, or, alternative, an "ok" signal in any form may be issued for user, such illumination of a (eg, green) light, etc. Illumination may change to red or other agreed color if inefficiency is found at some point in time. The monitoring system MT (or a different such device) may be used to monitor the said quantities derived from the contrast agent amount values, instead of or in addition to, the contrast agent amount values themselves.

In embodiments a quality control system QCS may be used that receives the metric and draws up a report of quality (contrast agent use) and forwards same by through an electronic communication channel to a recipient. The quality control system QCS may include or may cooperate/interface with the monitoring system/device MT.

The e-metric m may be processed by a graphics display generator GDG to generate the graphics display GD for display on display device DD. The graphics display may include a representation of the e-metric or quantities derivable therefrom. The e-metric may be displayed in the graphics display GD on the display device DD. One such optional graphics display GD may be configured to represent evolution of e-metric as will be described later below at Fig. 7, as one example herein of displaying such e-metric to so inform on CA usage.

The graphics display generated by generator GDG may further include at least a part of the input imagery, in addition to the output data such as the e-metric, or characteristics derived therefrom.

The computed contrast agent amount -metric values may be supplied to a control interface CI to control medical equipment. The control interface CI provides control signal m-OUT to control said equipment based on the computed e-metric on CA use efficiency. For example, the control signal m- m-OUT, may be provided as a feedback system to control stetting(s) of imager IA, or to the contrast agent release device RA, in order to control the manner in which contrast agent if released. A control loop may thus be released for operation of imager IA and or release device RA.

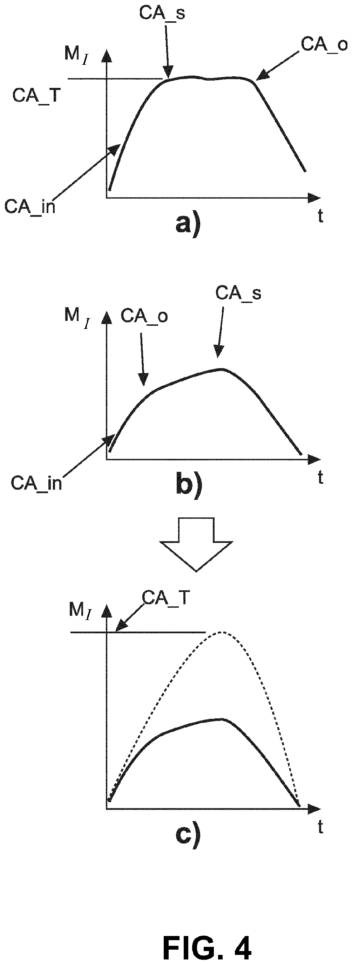

Various of the types of the efficiency metric, and/or related modes of operation of efficiency quantifier EQ are now illustrated below at Figs 4-7.

For computing the m-metric, in particular of types a)-c) or others, such operation may be based on (contrast agent) filling curve(s). Such curves CI, CI' represent CA amount values collected over the time, such as by integration as mentioned earlier above at (1) (2). The filling curves CI, CI' illustrate the typical phases as expected whilst the contrast agent bolus passes through the associated region of interest. There is a ramp up phase where contrast agent amount increases, followed by a plateau phase where the contrast agent amount saturates and remains relatively constant, and a follow-up drop-off phase where the contrast agent is washed out as it egresses the region of interest.

Reference is now made in more detail to Fig. 4 which illustrates various filling curves a), b), c). An interpolation method indicated by the arrow between curves b) and c) may be used by the efficiency quantifier EQ to compute the contrast agent amount (eg, mass), based on the spectral input imagery $\lambda$. In the illustrated filing curves, MI is the contrast agent amount axis, such as in mass, eg of Iodine, graphed vertically over time axis t. In the curves, section CA_in represent inflow of contrast agent or whilst contrast agent is released, section CA_s represents saturation, or when release is stopped, and section CA_o is the contrast agent outflow from ROI considered. CA_T is the total amount of contrast agent.

Specifically, Fig. 4 illustrates an interpolation which may be used by an analytic implementation of the efficiency quantifier EQ. Such interpolation may be used in particular for computing the efficiency metric mentioned above, In particular for types a)-c) e-metrics. The interpolation (shown as dashed curve in Fig. 4c)) may be needed if the full contrast agent dose is never detectable together in the imaged area ROI, possibly over one or plural frames. Eg, whilst contrast is still being released, part of the contrast agent is already flowing out of the FOV of the current image. Interpolation may then be used to estimate what the total injected amount was. In Fig. 4A) is the simple case, where it is assumed that contrast agent released stopped before outflow occurred. Thus, the plateau CA_T is the actual total amount of contrast agent. Any curve interpolation method, eg least squares, or any other may be used.

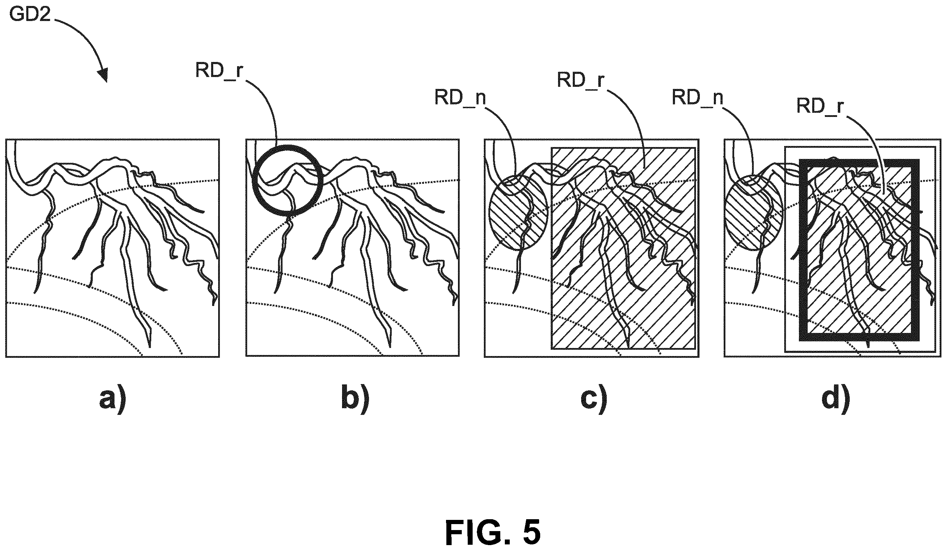

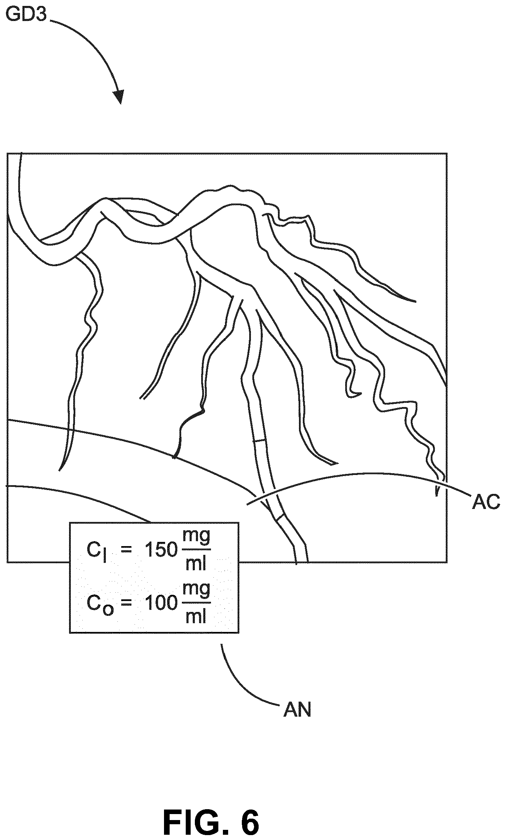

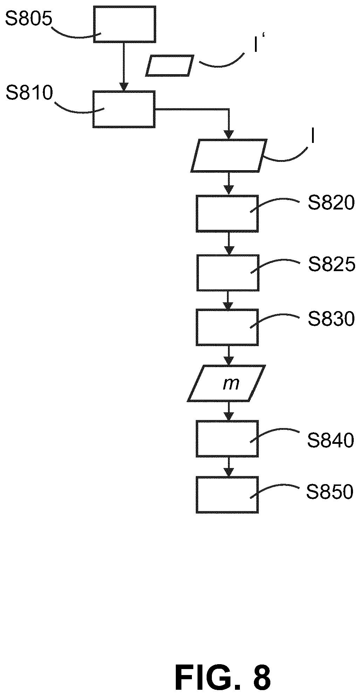

Fig. 5 illustrates operation of an embodiment of the efficiency quantifier EQ. This embodiment may be used preferably in analytical modelling for types a)-c). Fig. 6a) shows a spectral angiograph frame, whilst Figs 6b)-d) show the frame with region designators RD. Such region designators RD can also be used with benefit in the above-mentioned interpolation approach in Fig. 4 for estimating the contrast agent mass, on which the m-metric may be based. The estimator approach in Fig. 5 is based on the concept of designating region(s) of relevance RD_r and region(s) of non-relevance RD_n, preferably both relative to the ROI in one more input frames $\lambda$. Preferably, there is no overlap ( that is, their set theoretic intersection is empty) between region(s) of relevance RD_r and region(s) of non-relevance RD_n. The region of relevance RD_r may be equal to, or may be part of, the ROI, whilst the region of non-relevance RD_n may lie at least partly, such as fully outside, the region of relevance RD_r/ROI. By computing a respective contrast agent amount (eg, concentration), eg by integration (1),(2),in such relevant and non-relevant regions RD_r, RD_n, the above-mentioned e-metric m can be obtained. The e-metric m may be based on a ratio or difference (subtraction) of respective contrast agent amount in the two regions RD_r, n, or on any other suitable arithmetical combination, as will be described below in more detail at flow chart Fig. 8.

Fig. 5 may be understood as an illustration of the method of computing the said efficiency metric m based on the designate regions, but may also represent a graphics display GD2 that may be generated by graphics display generator GDG for display. Such graphics display may allow user to better verify operation of the system FS. The user may define the regions RD_r, RD_n manually through a UI (user interface - not shown), or the facilitator FS may suggest suitable segmentations for regions RD_r,n automatically or at least semi-automatically. In the latter case and as one such example thereof, the user merely provides seed-points from which the segmentations RD_r,n may be computed.

Fig. 6 shows yet another graphics display GD3 generatable herein by visualizer VIZ in cooperation with efficiency quantifier EQ. It includes a given angiographic spectral projection frame, or simply the underlying x-ray frame, with an associator component AC that indicates the region of interest ROI. The associator component AC associates the region of interest with an annotation box AN. The associator component AC may be rendered by visualizer VIZ for example as a bounding box/square/rectangle in the representation of GD3 - other geometrical renditions, such as circle/ellipse, others still are also envisaged. The associator component AC may be rendered as described above as an overlay that is positioned and sized to indicate the ROI. The annotation box AN may include information of the contrast agent amount at a given time. For example, the information in box AN may correspond to acquisition time of the X-ray projection frame displayed. Thus, the contrast agent amount value in bounding box AN of associator AC is associated with the acquisition time of the displayed frame, and relates to the amount of contrast agent value at the time the frame was acquired. Optionally, but preferably, the efficiency metric m is indicated in annotation box AN. The efficiency metric m may be associated with an optimal contrast agent amount value value C0 (eg, concentration), which may be displayed in addition to the current contrast agent amount value, to so better inform the user on the efficiency of the contrast agent-based operation. The associator component AC may have any shape or color, as desired. The two information items CI, C0 may together form the metric m in some embodiments. The difference $\Delta$ by which the current amount CI under- or overshoots the threshold C0 may or may not be indicated explicitly, as needed. Whilst the amounts of CA are shown in Fig. 6 in terms of concentration, this may not necessarily be so. The amounts may be display instead as mass amounts (eg, mg), or any other.

Fig. 7 shows yet another graphics display GD4 according to another embodiment, which may be understood as a dashboard and is generatable herein by visualizer VIZ in cooperation with efficiency quantifier EQ. The dashboard arrangement may be configured to consolidate some or all of the above-described quantities, including the contrast agent amount value(s).

One panel of graphics display GD4 (shown to the right) may represent the efficiency metric m over time t, as a time curve m(t). The current efficiency metric m may be displayed explicitly in addition or instead of such a curve, as shown in the lower left of graphics display GD4. The value m or curve m(t) is updated in graphics display GD4 as time progresses. In addition, or instead of, the current e-metric value m, an average efficiency metric $\bar{m}$ (eg, averaged over time) may be displayed.

The graphics display GD4 may include instead of m values/curve m(t), or in addition thereto, one or more panels S, M that describe other aspects of the contrast agent-based imaging procedure. For example, one panel S may indicate the source of the contrast agent used, such as the total volume administered. For example, the panel may indicate the amount S1 of manually administered contrast agent and/or the panel may indicate the amount S2 of automatically administered contrast agent, administered via the delivery apparatus DA, such as the pump.

Another optional panel M may include information on the imaging modality used, such as fluoroscopy M1, C-arm CT M2, or DSA (digital subtraction angiography) M3 for example.

It will be understood that in the various embodiments of the various graphics displays GDj described above, any given one is not necessarily at the exclusion of the other(s). Such graphics displays GDj may be displayed concurrently or in sequence, either all, or in any sub-combination, as desired by user. To this effect, the visualizer VIZ may be coupled to a user input interface to receive instructions which such (sub-)combination of the above-described graphics displays GDj is to be displayed on display device DD.

Having illustrated above some modes of computing e-metric by efficiency quantifier EQ, reference is now made to the flow chart of Fig. 8, where algorithmic aspects of computing such e-metrics in various embodiments are explained in more detail.

Thus, the flow chart represents a computer implemented method of computing an efficiency metric for contrast agent use during an intervention based on imaging data. It will be understood however, the method below may also constitute a teaching in its own rights, and is thus not necessarily tied to the system, use case, and graphic displays illustrated above at Figs 1-7, although the method may well be used in such connection.

At step S805, one or more projection raw images $\lambda$'E1, E2 at different spectra E1,E2 are acquired, based on using an imaging apparatus, preferably X-ray based, and configured for spectral imaging. Imaging hardware setups so configured may include imagers IA having multiple detectors, multiple X-ray sources operable to emit X-ray beams of different spectra, selectively filtering, kVp switching, etc. A bi-plane system may be used. Preferably, plural such projection images are so acquired, including at least one contrasted projection image. Optionally, at least one un-contrasted image is also acquired.

At step S810 the projection raw imagery $\lambda$' is processed by a spectral material decomposition algorithm such as described by R Alvarez & A Macovski in their US patent US 4,029 963, or in their paper "Energy-selective reconstructions in X-ray computerized tomography", published in "Physics in Medicine & Biology", vol 21 (5), pp733, (1976), or cognates thereof, in order to so obtain input spectral projection imagery $\lambda$ as explained above. In Alvarez et al, and related approaches, based on using a priori known attenuation coefficients of the contrast agent used and at least one other material or conglomerate of materials (eg, all but contrast agent, VNC), a system of simultaneous equations is set to and solved to obtain the amounts of CA.

Thus, the input spectral projection imagery is preferably based on contrasted multi-energy raw projection data, acquired thanks to the imager's multi-energy acquisition setup (eg dual layer), whilst there was contrast agent present in the FOV, or at least, in the ROI. The spectral projection imagery obtained by applying the spectral material decomposition algorithm is preferably VC, that is, any contrast is solely or predominantly due to (X-ray) attenuative contributions due to the contrast agent material present in the FOV. Thus, pixel values correspond to, and vary with, contrast agent material amount at each pixel location. Thus, pixel values are at the exclusion of contributions from other materials, or such contributions, if any, are negligible. Contrast agent may be, for example, Gadolinium, or Iodine, or any other. Specifically, such pixel value may represent as such, or by simple conversion, the amount of contrast agent that the beam XB passed through and to produce the respective pixel reading.

Alternatively, the spectral imagery may include virtual mono-energetic projection imagery, at an energy level that is known to yield good contrast for the contrast agent used, thanks, for example, to the contrast agent material's K-edge.

Such spectral projection imagery $\lambda$, either a still image or a time series of images (frames) over time, is received at step S820 as input imagery, and, based thereon, the efficiency metric m for contrast agent usage is computed at step S830. The efficiency metric m measures the efficiency of using the contrast agent for the medical imaging task/procedure/-protocol at issue. The computing S830 of e-metric may be based on integration eqs (1), (2) of spectral pixel (image) values, spatially and/or temporally as per eqs (1), (2) above. The spectral input imagery which may be so being integrated over is preferably a VC image or similar, with pixels values responsive to contrast agent presence at the exclusion or other materials, or at least with negligible contribution from such other materials. The spectral imagery being integrated over may be a DSA.

Any one or more of the above mentioned metric types of a)-f), or of two or more of the above-mentioned e-metric categories may be computed herein. Thus, the step S830 may be two-fold and may comprise at least two sub-steps: estimating/extracting of the CA amount such as by integration (1) or (2) and, based on the estimated amount, the metric is computed.

There may be an optional, prior to step S830, intermediate image conditioning step S825 where the input imagery $\lambda$ is any one or more of: (noise-)filtered and segmented. The computation at S830 may thus be confined to the so filtered and or segmented (identified) pixel values. The segmented pixels may be vessel pixels, as this is where the contrast agent can be expected to reside. Thus, not all pixels may need to be considered. Spatially and qualitatively, only pixels values within the medically/clinically relevant ROI or section thereof and those that represent contrast agent contribution are so considered in computing step S830. Thus, segmentation is configured to focus on the contrast agent contributions in the imagery from where contrast agent amount may be expected on purely anatomical ground, such as from vessels. The filtering may be configured to reduce noise and/or reduce pixel value contributions from materials other than contrast agent. If both filtering and segmenting is done, this can be done in any order, although noise filtering first and segmenting then may be preferred for better results. Either filtering or segmenting (but not necessarily both) may be done in some alternative embodiments, although implementing both, segmentation and filtering, may be preferred. Machine learning may be used for the segmenting, but analytical approaches, such as region-growing, snakes, or model-based segmentation (MBS) using modelling of internal and external abstract forces derived from in-image gradients are not excluded herein.

The efficiency metric m ("e-metric") computable at step S830 may be based purely on image information as per the spectral projection imagery, but may in addition use contextual data that pertains to the imaging procedure. Such contextual data $\kappa$ may include control parameters pertaining to operation of the imaging apparatus IA, used after or during the intervention/imaging operation. For example, computation may be based in addition on contextual parameters imaging parameters, such as imaging geometry, X-ray beam energy settings, etc.

In addition or instead, such contextual data $\kappa$ may include for example control parameters of the contrast agent release device RD. For example, such information may include any one of more of: the kind (material) of contrast agent that is released, its initial concentration when released, the total injected volume/mass, its injection rate, etc. For example, such control parameter may relate to the amount C0 of contrast agent released by the release device RD initially. This may be compared by thresholding or other, with the in-ROI contrast agent amount estimated by integration in the VC input image when computing metric at S830. The e-metric may then reflect this difference, if any. If there is a difference (beyond an error margin), this may be indicative of inefficient contrast agent use. Otherwise, the contrast agent use may be deemed efficient. Obtaining such control parameter on released CA from release device RD is one example for the pre-determined contrast agent amount. The contextual information $\kappa$ may further, or in addition, describes bio-characteristics of the patient PAT and this may inform how contrast agent propagates, distributes, etc, to so refine e-metric results.

At step S840 the so computed e-metric m may then be output for (further) processing at step S850. Such processing S850 may include displaying on a display device or any other, such as storing or controlling imaging supporting (or other) medical equipment, such as the imager IA itself, or the CA release device RD. Thus, a feedback loop may be effected in S850 to control imaging operation or CA release, so as to ensure the e-metric remains indicative of efficient CA use for example.

Depending on the nature of the computation at step S830, the metric m may be a single scalar value (binary or continuous), or may be itself a time series corresponding to the frames acquired during the imaging based invasive procedure.

Turning now in more detail to step S830, contrast agent mass MI extraction may be done by integration (1), (2), threshold-based segmentation or other. Extraction from a given frame of total (eg iodine) mass MI , may be done for a selected vessel segment. An UI may be used that allows user to make such selection.

Step S830 may include computing the above-described efficiency metric for assessing contrast agent usage relative to a given ROI as per metric of types a)-c) for example, or others.

The computing of such an efficiency metric m is now described in more detail, in particular with reference to metrics of type a)-c), or similar.

The concept of the efficiency metric in such types or others, may be based on the above defined regions of relevance RD_r =ROI= $A_R$ and region of non-relevance RD_n=$A_N$. such as introduced in Fig. 5 above. Such regions are a function of the imaging task at hand and are determined by the medical purpose and medical protocol. They may relate to a certain section of the coronaries. Areas outside the ROI= $A_R$ are deemed not relevant RD_n=$A_N$. However, the region(s) of non-relevance $A_N$ are in general not simply the set theoretic complement of $A_R$ but are in general adjacent, or neighboring areas of the $A_R$ of a given user-defined shape or size such as in Fig. 5, such as a circle, ellipse neighboring on the ROI. The efficiency metric m may describe the relative distribution of contrast agent mass in the two regions $A_R$, $A_N$. The respective regions $A_R$ , $A_N$ may not necessarily be topologically connected, but may be spread into separate regions without common boundary.

In such embodiments, step S830 may include the following sub-steps. The areas of procedural relevance $A_R$ and non-relevance $A_I$ are segmented for as was illustrated above in Fig. 5. The total contrast agent doses (masses) $M_{AR}$ and $M_{AN}$ in the areas $A_R$ and $A_N$, respectively, are computed preferably using the spectral contrast-agent only image VC thanks to the pixel values directly and quasiexclusively corresponding to contrast agent mass. This step may be based on integration (1), (2).

The efficiency metric m may be computed to vary with a deviation of the total Iodine amount (such as concentration) as estimated for the ROI=AR, from a predefined optimal concentration value $C_O$ . $C_O$ depends on the specific imaging task and the specific anatomy. Thus, the metric m may be reported as a function of |C0 - CI| or of (C0 - CI)2 or similar. In addition or instead or using amounts in concentration, any one or all of mass values $M_{AR}$ (mass in region of relevance), $M_{AN}$ (mass in region of non-relevance), $M_I$ (mass of CA) may be used on computing metric m. In addition or instead, metric m may be reported as a function of the ratio $M_{AR}/M_I$ or of $M_{AR}/M_{AN}$. The terms of "relevance" and "non-relevance" are a function of, and are defined by, the clinical imaging task at hand, the imaging protocol, etc.

In some embodiments, the e-metric m may be constructed so that it varies with the above mentioned deviation and/or with one or more of the said ratios. Specifically, and in an illustrative example, e-metric m may be reported as such a

$$m = M_{AR}/M_I \cdot \frac{1}{1+|C_I - C_O|}$$

combined metric as or a as any equivalent or any function thereof.

For contrast agent release over prolonged period of time ("long injections"), there may be contrast outflow OF (see Fig. 2 above) from the areas $A_R$ and $A_N$ as opposed to inflow at inflow IF (see Fig. 2 above). Thus, the total masses may not be reliably computed based on a single given projection frame. If the CA release period is relatively short, the masses as per $A_R$ and $A_N$ may be computed by integrating all image pixel values from the Iodine-only-image (more generally, from the VC image) in the respective areas $A_R$ and $A_N$. However, for longer contrast agent release periods, a part of the contrast agent may already be washed out of the imaged area whilst the CA-release operation is still ongoing. In other words, the total amount of administered contrast agent is not captured in a single image as mentioned. In this case either a filling curve interpolation or a ROI based flow interpolation approach may be used which are now describe in more detail.

The filling curve-based interpolation was illustrated above at Fig. 4c). The filling curve c describes the contrast agent (eg, Iodine) content as captured by the spectral image over time. Assuming a constant CA administration rate, the curve will increase constantly until the administration is stopped or until parts of the contrast agent starts to leave via OF the imaged area ROI. As illustrated by the dashed curve in Fig. 4c), the total contrast agent dose can be extracted from an interpolated filling curve. For example, estimator EQ may approximate an inflow rate at inflow IF and an outflow rate at outflow OF. E.g. such may be assumed constant, or may be fitted by another function. The stop time of CA release is estimated by whichever means, or is received as a signal from CA release apparatus RA. The approximated outflow amount may then be added to the measured curve in the time period when inflow and outflow overlap. This may be conceptualized by imaging the middle portion of a relatively long pipe, with a long bolus passing thereto.

The ROI based flow interpolation may use as inflow regions IF (see Fig. 2 above) one more (concentric) regions of interest around a guide catheter for example, to derive a contrast agent inflow value as illustrated in Fig. 6. The total contrast agent dose may then be calculated as the integrated contrast inflow over the contrast agent administration time, preferably taking into account (eliminating) attenuation contributions of the guide catheter, whose material composition and shape, etc, is assumed known, and through which the contrast bolus is administered when computing the initial contrast agent amount (eg, concentration). Alternatively, a contrast outflow OF from the imaged area is estimated from concentric ROIs at the border of the imaged area. The total contrast agent dose is then calculated at the end of administration as the sum of contrast agent in the image and the integrated contrast outflow from the imaged area. The outflow estimation can further rely on vessel segmentations of vessels such as collaterals that emanate from the imaged area.

The above-described mass interpolation approach, filling curve or ROI based, may also be used for determining the contrast agent mass, instead of concentration.

In embodiments, the system may include a user interface (not shown), configured to allows a user to provide manual input of total contrast agent dose to correct possible errors in the automated calculation of $M_I$, $M_{AR}$, and $M_{AI}$, or metric m. A cross-check may be done by using the relation $M_I = M_{AI} + M_{AR}$ to validate reliability of the computations.

With knowledge of the total contrast amount used during the intervention (as supplied by automatically by pump RA or manually), the relative contrast agent amount used for imaging / image guidance can be determined by analyzing some or all images / image sequences / C-arm CT images throughout the intervention to arrive at a time series of metric values m as was illustrated in Fig. 7. This will result in a quality measure for a performed intervention. The metric m can be analyzed for the complete intervention or for parts thereof such as for suitable periods steps during the whole intervention (pre-treatment / during treatment / post treatment). The dashboard Fig. 7 or similar, may be configured to illustrate metric mt over time and can serve as a quality measure for procedures carried out in a single lab / across multiple labs / across multiple hospitals, etc, as required.

As to type c) metric or others, in some embodiments, the CA amount may be expressed as a concentration rather than absolute mass. Thus, in step S830, a ratio MI/VV may be formed between extracted mass MI as per the spectral input image λ, and an obtained lumen volume VV to quantify the contrast agent concentration CI. The lumen volume dimension L may be estimated from a vessel model, or by using a C-arm or bi-plane system and acquiring projection along different projection directions. Shape primitives, such as cylinders may be used to estimate volume. More specifically, step S830 for c)-type e-metric may include segmenting for an outline of at least one contrast filled vessel segment in the area of procedural relevance RD_r. A local diameter of vessel may be determined from a segmentation measurement. An approximate vessel lumen V may be derived using a geometry model (primitives), such as an assumption of circular cross

$$C_I = \frac{M_V}{V}$$

sections. As earlier observed, the CA concentration may be computed as , where $M_V$ is the integrated Iodine content in the segmented vessel area (see Fig. 6). In order to compute the metric m, local Iodine concentration $C_I$ may be compared to a predefined optimal value $C_O$. The predefined optimal value $C_O$ may depend on the specific imaging task and the specific anatomy.

Turning now to d) -type e-metrics or similar, for any recorded sequence λt with contrast agent detected in the VC image(s), the number of frames without any contrast agent amount (or less than a threshold) are counted in step S830. The count may represent a measure for unnecessary X-ray exposures. Step S830 may further include calculating, by integration or other, the amount of contrast agent that is already present at the first frame in the sequence. If the amount of contrast agent in the first frame is above a threshold, the sequence was initiated too late. In addition or instead, from a filling curve (see Fig. 4) it can be determined if the end of the sequence was in the drop-off phase of the curve, i.e. in the outflow phase. If this is not the case, the run was not recorded long enough.

Turning now to e) -type e-metrics or similar, for any sequence λt in which contrast agent amount is detected in the VC image(s), the imager's parameters/settings/protocols etc used are stored. Based on the total amount of contrast agent measured in the sequence by using (2), the appropriate use of the protocol is evaluated in step S830. For example, a smaller bolus can be expected to be recorded under fluoroscopy, whilst a larger bolus can be expected to be recorded with an angiography protocol. A rotational imaging run should have sufficient contrast filling throughout the whole sequence.

Thus, a binary m-metric may be computed by using a CA amount threshold for the given imaging protocol (fluoro, angio, rotational). Alternatively, m-metric may be provided as an amount number, such as x ml of iodine used during rotational, y ml used during fluoroscopy, etc. The total over time contrast agent amount is then compared against the correct threshold, to output e-metric an indication on whether CA use is efficient or not, based on thresholding.

Turning now to f) -type e-metrics or similar, step S83 may include segmenting for the vessel to obtain a vessel map image. A binary segmentation for "vessel" / "no vessel" of all image pixels, or for image pixels in a selected subset (that corresponds to the anatomy of interest), may be done. Such segmentation can be done through filtering or by machine learning. The e-metric may then be computed as a projective vessel overlap (vessel that appear overlapped in projection view) metric: the highest integrated (as per (1)) CA amount may indicate the frame(s) with best contrast filling. A high combination of integrated CA amount and integrated vessel class pixels may indicate frame(s) with best contrast filling and low vessel overlap. A high integrated iodine amount and low integration of vessel class pixels indicates good contrast filling, but strong vessel overlap or foreshortening. Thus, more generally, the ratio between contrast amount and number of pixels representative of in-vessel location may be formed to compute e-metric. Again, thresholding of the ratios may be used to conclude efficiency / non-efficiency. In general, an image with little vessel overlap would typically be better, as the vessel is better visible individually, and is not occluded by other vessels. The overlap concept could be focused around know specific pathologies. E.g. an aneurism which should not overlap with large vessels during treatment so as to better image the anatomy ROI.

Instead of computing e-metric in an analytical setup as described above, machine leaning model M such an artificial neural network (NN) or other may be used. Training data of prior imagery from historical examinations may be used. Such may be queried from databases, such as PACS, HIS or other. A clinical expert may be used to annotate or label m' the data by assigning training e-metrics as targets, based on the clinical's experts understanding on whether or not contrast agent was used efficiently as per the respective historical angiographic imagery. The labelling may also be derived from information held in associated patent records, such as information on amount of contrast agent used, or notes/comments made by a reviewer or quality checker, auditor, etc on contrast agent use, etc. A regression or classifier model M may be used, such as the said NN (such as a convolutional NN (CNN)), or an SVM, decision trees, etc may be used. A training algorithm may be used to adjust parameters of the model based on the training data. Training may be driven by an objective function. Model parameters may be adjusted so as to improve an objective function, such as cost function. Cost function F may measure a deviation, if any, between model output $M(x)$ based on training imagery $x$, and the target (expert label $y=m'$). The model parameters $\theta$ may be adjusted to that training model output matches target: $\text{argmin}\theta\, F\theta(D[M\theta\,(x), y])$, with $D(.,.)$ a suitable distance function to measure the said deviation. The training algorithm may be based on gradients of the cost function, such gradient descent, stochastic gradient, etc. Backpropagation techniques may be used.

Applications outside the medical field, such as use of a dye in assessing flow characteristics in inaccessible subterranean drainage systems, rivers, etc, are also envisaged herein. In some ex-medical applications such dyes in river systems may cause environmental harm, and the proposed setup allows more efficient use of such dye to better protect the environment.

Other applications of the principles described herein may include the engineering domain, such as a dye-based assessment or maintenance of hydraulic or cooling systems in vehicles, power plants, etc. In such applications, excess contamination with dye may be undesirable due to effects on the machine parts or due to costs of the dye, etc. The proposed setup fosters frugal and yet, for the task effective, use of flow-entrained contrasts, dye, etc in various such or other applications.

The components of system FS may be implemented as one or more software modules, run on one or more general-purpose processing units PU such as a workstation associated with the imager IA, or on a server computer associated with a group of imagers. The workstation or other data processing unit(s) PU may be part of the imaging apparatus IA.

Alternatively, some or all components of system FS may be arranged in hardware such as a suitably programmed microcontroller or microprocessor, such an FPGA (field-programmable-gate-array) or as a hardwired IC chip, an application specific integrated circuitry (ASIC), integrated into the imaging system FS. In a further embodiment still, system FS may be implemented in both, partly in software and partly in hardware.

The different components of system FS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters, or of any other alphanumeric combination.

**Claims**

1. System (FS) for quantifying efficiency of use of contrast agent in an imaging procedure, comprising:

    an input interface (IN) through which is receivable input data comprising spectral projection imagery ($\lambda$) of an object (PAT) obtainable by a spectral X-ray imaging apparatus (IA) in an imaging procedure at at least one field of view, FOV, with contrast agent (CA) present at least at times in the FOV; and
    an efficiency quantifier (EQ) configured to estimate an amount of the contrast agent from the spectral projection imagery and compute a metric (m) indicative of said efficiency of use of contrast agent, based on the estimated amount.

2. System of claim 1, wherein the metric is based on the total amount of contrast agent used in a part or the whole of the imaging procedure.

3. System of any claim 1 or 2, wherein estimating the amount of the contrast agent includes integrating, spatially and/or temporally, over pixel values in one or more contrast agent-only-images derived from the spectral projection imagery acquired of the region of interest.

4. System of any one of preceding claims, wherein the metric is based on the amount used locally in at least one subset of the FOV, or in the whole of the FOV, as captured in the spectral projection imagery.

5. System of any one of preceding claims, wherein the metric is based on comparing the said amount against a predetermined amount of contrast agent, the predetermined amount being provided by a contrast agent administering device configured to measure an amount of contrast agent being administered.

6. System of any one of preceding claims, wherein the efficiency metric (m) is computed based on a segmented portion (s) in the spectral imagery, and the said amount is estimable relative to the said segmented portion.

7. System of claim 6, wherein segmenting the segmented portion distinguishes between a first region of interest (ROI, RG_R) for the current imaging procedure and a second region different from the first region (RG_I), and wherein the efficiency metric is based on a first contrast agent amount in the first region of interest and a second contrast agent amount in the second region of interest.

8. System of any one of the preceding claims, wherein the metric is computed based on a filing curve that traces the amount of contrast agent over time.

9. System of any one of the preceding claims, wherein the contrast agent is passable through a conduit (CN) being at least a part of a vascular, urinary or lymphatic system of a mammal, such as a human patient.

10. System of any one of the previous claims, wherein the spectral X-ray imaging apparatus is a C-arm type X-ray apparatus comprising a dual-layer X-ray detector.

11. System of any one of the previous claims, wherein the computed metric (m) is provided for processing, wherein the processing includes any one or more of: i) displaying on a display device an indication of the metric, ii) storing same in a data storage, iii) controlling operation of medical equipment, and iv) processing by a quality control system (QCS).

12. An imaging arrangement (MIA) comprising the system of any one of the previous claims, and including the spectral X-ray imaging apparatus.

13. Method of image processing, comprising:

   receiving (S820) input data comprising spectral projection imagery ($\lambda$) of an object (PAT) obtainable by a spectral X-ray imagining apparatus (IA) in an imaging procedure at at least one field of view, FOV, with contrast agent (CA) present at least at times in the FOV; and
   computing (S830) a metric (m) indicative of said efficiency of use of said contrast agent, including:
   estimating an amount of the contrast agent from the spectral projection imagery.

14. A computer program element, which, when being executed by at least one processing unit (FS), is adapted to cause the processing unit to perform the method as per claim 13.

15. At least one computer readable medium having stored thereon the program element of claim 14.

**FIG. 1**

**FIG. 2**

FIG. 3

FIG. 4

**FIG. 5**

EP 4 483 810 A1

GD3

AC

$$C_I = 150\frac{mg}{ml}$$

$$C_O = 100\frac{mg}{ml}$$

AN

# FIG. 6

GD4

m(t)

S

S1 : 42 ml
S2 : 20 ml

M1 : 12 ml
M2 : 30 ml
M3 : 10 ml

M

$m$ : 83.9%
$\overline{m}$ : 78.5%

$m$
100,00
90,00
80,00
70,00
60,00
50,00
40,00
30,00
20,00
10,00
0,00

1  2  3  4  5  6  7  8  9 10 11  t

# FIG. 7

S805

I'

S810

I

S820

S825

S830

m

S840

S850

# FIG. 8

| | Europäisches Patentamt European Patent Office Office européen des brevets | **EUROPEAN SEARCH REPORT** | Application Number EP 23 18 2095 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 10 740 932 B2 (KONINKLIJKE PHILIPS NV [NL]) 11 August 2020 (2020-08-11)<br>* abstract *<br>* column 3, line 23 - column 7, line 19 *<br>* figure 1 *<br>----- | 1-6,<br>10-15<br>7-9 | INV.<br>A61B6/00 |
| X<br><br>Y<br>A | US 2013/261441 A1 (DAS BIPUL [IN] ET AL) 3 October 2013 (2013-10-03)<br>* abstract *<br>* paragraph [0016] - paragraph [0029] *<br>* figure 3 *<br>----- | 1,13-15<br><br>7-9<br>2-6,<br>10-12 | |
| Y | ERNST KLOTZ ET AL: "Perfusion measurements of the brain: using dynamic CT for the quantitative assessment of cerebral ischemia in acute stroke",<br>EUROPEAN JOURNAL OF RADIOLOGY, ELSEVIER SCIENCE, NL,<br>vol. 30, no. 3, 1 June 1999 (1999-06-01),<br>pages 170-184, XP002621724,<br>ISSN: 0720-048X, DOI:<br>10.1016/S0720-048X(99)00009-1<br>[retrieved on 1999-07-16]<br>* abstract *<br>* section 2. Methods and Material *<br>----- | 7-9 | TECHNICAL FIELDS SEARCHED (IPC)<br><br>A61B |

The present search report has been drawn up for all claims

| Place of search | Date of completion of the search | Examiner |
|---|---|---|
| Munich | 26 October 2023 | Montes, Pau |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 483 810 A1**

## ANNEX TO THE EUROPEAN SEARCH REPORT
## ON EUROPEAN PATENT APPLICATION NO.

EP 23 18 2095

This annex lists the patent family members relating to the patent documents cited in  the above-mentioned European search report.
The members are as contained in the European Patent Office EDP file on
The European Patent Office is in no way liable for these particulars which are merely given for the purpose of information.

26-10-2023

| Patent document cited in search report | | | Publication date | Patent family member(s) | | | Publication date |
|---|---|---|---|---|---|---|---|
| US 10740932 | B2 | | 11-08-2020 | CN | 107257991 | A | 17-10-2017 |
| | | | | EP | 3262612 | A1 | 03-01-2018 |
| | | | | JP | 6559791 | B2 | 14-08-2019 |
| | | | | JP | 2018506366 | A | 08-03-2018 |
| | | | | US | 2018040146 | A1 | 08-02-2018 |
| | | | | WO | 2016135044 | A1 | 01-09-2016 |
| US 2013261441 | A1 | | 03-10-2013 | CN | 103356206 | A | 23-10-2013 |
| | | | | JP | 6139207 | B2 | 31-05-2017 |
| | | | | JP | 2013202414 | A | 07-10-2013 |
| | | | | US | 2013261441 | A1 | 03-10-2013 |

EPO FORM P0459

For more details about this annex : see Official Journal of the European Patent Office, No. 12/82

## REFERENCES CITED IN THE DESCRIPTION

**Patent documents cited in the description**

- US 4029963 A

**Non-patent literature cited in the description**

- Energy-selective reconstructions in X-ray computer-ized tomography. *Physics in Medicine & Biology*, 1976, vol. 21 (5), 733